# EUROPÄISCHE PATENTSCHRIFT

(11) **EP 4 300 115 B1**
(45) Veröffentlichungstag und Bekanntmachung des Hinweises auf die Patenterteilung: **01.01.2025**
(21) Anmeldenummer: 22182181.2
(22) Anmeldetag: 30.06.2022
(51) Int. Cl.: G01R 33/28, G01R 33/36, G01R 33/48, A61B 6/00, A61B 6/03, A61B 5/055, H02K 37/00, A61B 5/00, A61B 6/04

(54) **MR-PET-VORRICHTUNG MIT EINER EINSTELLEINHEIT ZU EINER EINSTELLUNG EINER POSITION EINES STECKVERBINDUNGSELEMENTS SOWIE EIN VERFAHREN ZU EINEM EINSTELLEN EINER POSITION EINES STECKVERBINDUNGSELEMENTS**
MR-PET DEVICE WITH AN ADJUSTMENT UNIT FOR SETTING A POSITION OF A PLUG CONNECTION ELEMENT AND A METHOD FOR ADJUSTING A POSITION OF A PLUG CONNECTION ELEMENT
DISPOSITIF RM-PET DOTÉ D'UNE UNITÉ DE RÉGLAGE DESTINÉE AU RÉGLAGE D'UNE POSITION D'UN ÉLÉMENT CONNECTEUR ENFICHABLE, AINSI QUE PROCÉDÉ DE RÉGLAGE D'UNE POSITION D'UN ÉLÉMENT CONNECTEUR ENFICHABLE

(43) Veröffentlichungstag der Anmeldung: 03.01.2024
(73) Patentinhaber: Siemens Healthineers AG, 91301 Forchheim (DE)
(72) Erfinder: Fenchel, Matthias, 91054 Erlangen (DE); Sukkau, Johann, 91074 Herzogenaurach (DE)
(74) Vertreter: Siemens Healthineers Patent Attorneys

(56) Entgegenhaltungen:
- DE-U1- 202021 103 323
- US-A1- 2008 030 195

## Beschreibung

Die vorliegende Erfindung betrifft eine MR-PET-Vorrichtung mit einem Patiententisch und einer Steckverbindungseinheit, die zumindest ein Steckverbindungselement, das am Patiententisch angeordnet ist, aufweist, wobei das zumindest eine Steckverbindungselement dazu ausgebildet ist, mit einem korrespondierenden Steckverbindungselement einer externen Vorrichtung eine lösbare Verbindung einzugehen. Des Weiteren geht die vorliegende Erfindung aus von einem Verfahren zu einem automatischen Einstellen einer Position zumindest eines Steckverbindungselements an einem Patiententisch.

Es sind Magnetresonanz-Positronen-Emissions-Tomographie-Vorrichtungen (MR-PET-Vorrichtungen) bekannt, beispielsweise aus US20060293580A1, welche eine kombinierte und simultane Aufnahme von Magnetresonanz-Signalen und PET-Signalen ermöglichen. Befinden sich im Strahlengang von PET-Photonen jedoch Hardwarekomponenten, verursacht dies eine Signalschwächung. Es gibt verschiedene Ansätze eine solche Signalschwächung im Rahmen einer sogenannten Schwächungskorrektur zu berücksichtigen. Allerdings gelingt dies in der Regel nicht vollkommen, so dass es dennoch zu Bildartefakten in den rekonstruierten PET-Abbildungen kommen kann.

Aus DE 20 2021 103 323 U1 ist ein MR-PET-Gerät mit einem beweglichen Steckverbindungsteil bekannt. Hierbei kann mit Hilfe einer Positionierungsvorrichtung das Steckverbindungsteil von einer ersten Position in zumindest eine weitere Position bewegt werden.

Der vorliegenden Erfindung liegt insbesondere die Aufgabe zugrunde, etwaige Bildartefakte in den rekonstruierten PET-Abbildungen zu reduzieren. Die Aufgabe wird durch die Merkmale der unabhängigen Ansprüche gelöst. Vorteilhafte Ausgestaltungen sind in den Unteransprüchen beschrieben.

Die Erfindung geht aus von einer MR-PET-Vorrichtung mit einem Patiententisch und einer Steckverbindungseinheit, die zumindest ein Steckverbindungselement, das am Patiententisch angeordnet ist, aufweist, wobei das zumindest eine Steckverbindungselement dazu ausgebildet ist, mit einem korrespondierendem Steckverbindungselement einer externen Vorrichtung eine lösbare Verbindung einzugehen. Erfindungsgemäß ist eine Position des zumindest einen Steckverbindungselements einstellbar, wobei die Steckverbindungseinheit eine Einstelleinheit aufweist für eine selbsttätige Einstellung einer Zielposition des zumindest einen Steckverbindungselements am Patiententisch. Die Einstelleinheit weist eine Motoreinheit, die mit dem zumindest einen Steckverbindungselement verbunden ist, ein Führungselement, in dem das zumindest eine Steckverbindungselement der Steckverbindungseinheit bewegbar gelagert ist, ein Übertragungselement, das zu einer Einstellung von zumindest zwei unterschiedlichen Zielpositionen des zumindest einen Steckverbindungselements innerhalb des Führungselements ausgebildet ist, und einen Kraftsensor auf zur Erfassung einer auf das Übertragungselement wirkenden Zugkraft
Die MR-PET-Vorrichtung (Magnetresonanz-Positronen-Emissions-Tomographie-Vorrichtung) umfasst eine Magnetresonanzvorrichtung und eine Positronen-Emissions-Tomographie-Vorrichtung (PET-Vorrichtung). Die Magnetresonanzvorrichtung umfasst eine Scannereinheit mit einem Grundmagneten, einer Gradientenspuleneinheit und einer Hochfrequenzantenneneinheit und ist zu einer Erfassung von Magnetresonanzsignalen eines zu untersuchenden Bereichs eines Patienten ausgebildet. Die PET-Vorrichtung weist Positronen-Emissions-Tomographie-Detektormodule (PET-Detektormodule) mit Szintillationsdetektorelementen und Photodioden auf. Insbesondere ist die PET-Vorrichtung zur Erfassung von PET-Daten ausgebildet.

Die MR-PET-Vorrichtung umfasst bevorzugt eine Patientenlagerungsvorrichtung mit einem bewegbaren Patiententisch. Ein Patient, insbesondere ein zu untersuchender Bereich des Patienten, wird bevorzugt mittels des bewegbaren Patiententischs in einen Patientenaufnahmebereich, insbesondere in ein Field of View (FOV), der MR-PET-Vorrichtung für eine MR-PET-Untersuchung eingefahren und/oder positioniert. Der Patiententisch weist hierzu einen Lagerungsbereich zur Lagerung und/oder zur Positionierung des Patienten auf.

Für eine MR-PET-Untersuchung, insbesondere für eine MR-Untersuchung werden häufig externe Vorrichtungen, insbesondere Zubehöreinheiten, wie beispielsweise lokale Hochfrequenzspulen, am Patienten und/oder am Patiententisch angeordnet. Eine derartige Hochfrequenzspule kann beispielsweise eine Kopf-Hochfrequenzspule oder einer Rücken-Hochfrequenzspule usw. sein. Für eine Verbindung der externen Vorrichtung, insbesondere der Zubehöreinheit, mit der MR-PET-Vorrichtung, insbesondere einer Auswerteeinheit der MR-PET-Vorrichtung, ist am Patiententisch eine Steckverbindungseinheit mit zumindest einem Steckverbindungselement angeordnet. Die Steckverbindungseinheit kann auch zwei oder mehr Steckverbindungselemente umfassen. Für eine Verbindung der externen Vorrichtung, insbesondere der Zubehöreinheit, mit der MR-PET-Vorrichtung, insbesondere einer Auswerteeinheit und/oder Steuereinheit der MR-PET-Vorrichtung, weist auch die externe Vorrichtung, insbesondere die Zubehöreinheit, ein zu dem zumindest einen Steckverbindungselement der Steckverbindungseinheit korrespondierendes Steckverbindungselement auf. Dabei kann das zumindest eine Steckverbindungselement der Steckverbindungseinheit eine Steckerbuchse und das dazu korrespondierende Steckverbindungselement der externen Vorrichtung, insbesondere der Zubehöreinheit, einen dazu passenden Stecker umfassen. Insbesondere sind die beiden Steckverbindungselemente bevorzugt Teile einer gemeinsamen Steckverbindung.

Die Steckverbindungselemente können insbesondere einen oder mehrere elektrische und/oder optische Kontakte umfassen zur Übertragung von, insbesondere elektrischen und/oder optischen, Signalen und/oder, insbesondere elektrische und/oder optische, Leistung. Über die Steckverbindungseinheit, insbesondere das zumindest eine Steckverbindungselement, und dem dazu korrespondierenden Steckverbindungselement der externen Vorrichtung erfolgt ein Datenaustausch zwischen der externen Vorrichtung, beispielsweise der lokalen Hochfrequenzspule, und einer Auswerteeinheit und/oder Steuereinheit der MR-PET-Vorrichtung. Dieser Datenaustausch kann beispielsweise erfasste Magnetresonanzdaten und/oder Steuerdaten zur Ansteuerung der externen Vorrichtung usw. umfassen.

Unter einer lösbaren Verbindung zwischen dem zumindest einen Steckverbindungselement der Steckverbindungseinheit und dem dazu korrespondierenden Steckverbindungselement der externen Vorrichtung soll dabei eine Verbindung verstanden werden, die durch trennen der beiden Steckverbindungselemente getrennt und/oder aufgehoben ist. Durch ein erneutes Verbinden der beiden Steckverbindungselemente kann die lösbare Verbindung wieder hergestellt werden.

Durch die Anordnung der externen Vorrichtung am Patienten und/oder am Patiententisch für eine MR-PET-Untersuchung können sich im Strahlengang der PET-Photonen zusätzliches Material und/oder Gegenstände, insbesondere Teilbereiche der externen Vorrichtung, befinden, die zu einer Schwächung des PET-Signals führen können. Um diese Gegenstände, insbesondere Teilbereiche der externen Vorrichtung, außerhalb des Strahlengangs der PET-Photonen anzuordnen, ist die Position des zumindest einen Steckverbindungselements der Steckverbindungseinheit am Patiententisch einstellbar ausgebildet. Bevorzugt weist hierbei die Steckverbindungseinheit ein Führungselement, beispielsweise eine Führungsschiene und/oder eine Führungsrinne, auf, in der das zumindest eine Steckverbindungselement bewegbar angeordnet ist, so dass das zumindest eine Steckverbindungselement je nach Untersuchungsart und/oder zu untersuchenden Bereich des Patienten aus dem FOV der MR-PET-Vorrichtung, insbesondere aus dem FOV der PET-Vorrichtung, für eine MR-PET-Messung heraus bewegt werden kann. Bevorzugt wird hierbei das zumindest eine Steckverbindungselement der Steckverbindungseinheit in eine Zielposition bewegt, wobei die Zielposition außerhalb des FOVs der MR-PET-Vorrichtung, insbesondere einem FOV der PET-Vorrichtung, angeordnet ist.

Des Weiteren weist die Steckverbindungseinheit eine Einstelleinheit auf, die zu einer selbsttätigen und/oder automatischen Einstellung der Zielposition des zumindest einen Steckerbindungselements am Patiententisch ausgelegt ist. Die Einstelleinheit weist hierzu eine Motoreinheit zu einer Generierung eines Antriebsmoments für eine Bewegung des zumindest einen Steckverbindungselements in die Zielposition auf.

Zudem kann die Einstelleinheit auch eine Steuereinheit und/oder Recheneinheit zu einer Steuerung der selbsttätigen und/oder automatischen Einstellung der Zielposition des zumindest einen Steckverbindungselements am Patiententisch aufweisen. Die Einstelleinheit kann dabei eine aktuelle Position des zumindest einen Steckverbindungselements und/oder eine Position des Patiententischs und/oder ein FOV der PET-Messung und/oder weitere Parameter bei der Einstellung der Zielposition des zumindest einen Steckverbindungselements berücksichtigen.

Durch die Erfindung kann vorteilhaft das Steckverbindungselement besonders zeitsparend in seine Zielposition bewegt werden und damit außerhalb des FOV der PET-Vorrichtung angeordnet und/oder positioniert werden. Damit einhergehend können unerwünschte Artefakte und/oder Störeinflüsse in den PET-Bilddaten minimiert werden und auch eine Bildqualität vorteilhaft erhöht werden.

Gegenüber einer manuellen Einstellung, die vor einem Einfahren des Patienten in den Patientenaufnahmebereich erfolgt, bei der der Benutzer die Zielposition nur grob abschätzen kann, kann hierbei die automatische Einstellung der Zielposition des zumindest einen Steckverbindungselements auch erst erfolgen, wenn der zu untersuchende Bereich bereits im FOV der MR-PET-Vorrichtung angeordnet ist. Hingegen kann es bei einer manuellen Einstellung der Zielposition des zumindest einen Steckverbindungselements vorkommen, dass hierzu der Patient mehrmals aus dem Patientenaufnahmebereich der MR-PET-Vorrichtung hinein und wieder herausgefahren wird und jedes Mal die Position des zumindest einen Steckverbindungselements neu eingestellt werden muss, bis diese nicht mehr in dem FOV insbesondere der PET-Vorrichtung angeordnet ist, was jedoch sehr zeitaufwendig ist.

Ein weiterer Vorteil ist, dass aufgrund der zeitsparenden Positionierung des zumindest einen Steckerelements ein Zerfall eines Tracers und/oder eines Radiopharmakons bereits vor Beginn der MR-PET-Untersuchung verhindert wird und damit eine zusätzlichen Strahlenexposition im Patienten vermieden wird.

Erfindungsgemäß kann es vorgesehen sein, dass die Einstelleinheit eine Motoreinheit aufweist, die mit dem zumindest einen Steckverbindungselement verbunden ist. Die Motoreinheit ist hierbei zu einer Generierung eines Antriebsmoment für eine Bewegung des zumindest einen Steckverbindungselements, insbesondere innerhalb eines Führungselements der Einstelleinheit, ausgebildet. Bevorzugt ist die Motoreinheit magnetresonanzkompatibel ausgebildet, so dass während einer Generierung eines Antriebsmoments eine unerwünschte Interaktion zwischen der Motoreinheit und der Magnetresonanzvorrichtung vorteilhaft verhindert werden kann. Beispielsweise kann die Motoreinheit einen magnetresonanzkompatiblen Schrittmotor, wie er in der Patentanmeldung DE 10 2020 211 326 A1 oder der Patentanmeldung DE 10 2020 211 327 A1 beschrieben ist, umfassen. Alternativ oder zusätzlich kann die Motoreinheit auch einen pneumatische Schrittmotor und/oder einen Piezo-Schrittmotor und/oder elektrischen Schrittmotor umfassen. Die Einstelleinheit, insbesondere die Motoreinheit, weist zudem ein Übertragungselement auf, das das von der Motoreinheit generierte Antriebsmoment auf das zumindest eine Steckverbindungselement überträgt bzw. die Motoreinheit mit dem zumindest einen Steckverbindungselement verbindet. Das Übertragungselement umfasst bevorzugt einen Seilzug für eine besonders exakte Einstellung einer Position des zumindest einen Steckverbindungselements. Alternativ kann das Übertragungselement auch einen Riemen und/oder eine Kette und/oder eine Welle usw. umfassen. Hierdurch kann auf konstruktiv einfache Art und Weise ein Antriebsmoment für eine Bewegung, insbesondere eine automatische und/oder selbsttätige Positionierung, des zumindest einen Steckverbindungselements der Steckverbindungseinheit bereitgestellt werden.

In einer vorteilhaften Weiterbildung der erfindungsgemäßen MR-PET-Vorrichtung kann es vorgesehen sein, dass die Motoreinheit an einem Fußende des Patiententischs angeordnet ist. Bevorzugt umfasst hierbei die Motoreinheit einen pneumatische Schrittmotor und/oder einen Piezo-Schrittmotor und/oder elektrischen Schrittmotor. Das Fußende des Patiententischs umfasst dabei dasjenige Ende des Patiententischs, das in einem hinteren Bereich des Patiententischs in Einfahrrichtung angeordnet ist und/oder während eines Einfahrens des Patiententischs in den Patientenaufnahmebereich als letztes in den Patientenaufnahmebereich eingefahren wird. Während einer MR-PET-Untersuchung ragt das Fußende des Patiententischs meist aus dem Patientenaufnahmebereich heraus. Dagegen umfasst ein Kopfende des Patiententischs dabei dasjenige Ende des Patiententischs, das in einem vorderen Bereich des Patiententischs in Einfahrrichtung angeordnet ist und/oder während eines Einfahrens des Patiententischs in den Patientenaufnahmebereich als erstes in den Patientenaufnahmebereich eingefahren wird. Derart kann eine vorteilhafte Anordnung der Motoreinheit erreicht werden, bei der eine unerwünschte Interaktion mit der Magnetresonanzvorrichtung verhindert werden kann.

In einer vorteilhaften Weiterbildung der erfindungsgemäßen MR-PET-Vorrichtung kann es vorgesehen sein, dass die Einstelleinheit ein Führungselement aufweist, in dem das zumindest eine Steckverbindungselement der Steckverbindungseinheit bewegbar gelagert ist, wobei die Motoreinheit ein Übertragungselement aufweist, das zu einem Verschieben des zumindest einen Steckverbindungselements innerhalb des Führungselements um mindestens 1,5 mm und um maximal 40 cm innerhalb des Führungselements ausgebildet ist. Das Führungselement umfasst bevorzugt eine Führungsschiene und/oder eine Führungsrinne, in der das zumindest eine Steckverbindungselement bewegbar, insbesondere in eine Richtung bewegbar, vorzugsweise in Längsrichtung des Patiententischs, gelagert ist. Bevorzugt ist das Führungselement in einem seitlichen Bereich des Patiententischs neben einem Lagerungsbereich zur Lagerung des Patienten angeordnet. Das Führungselement weist bevorzugt ein Signalübertragungselement auf, das in jeder einstellbaren Position des zumindest einen Steckverbindungselements eine Übertragung und/oder einen Austausch von Signalen und/oder Daten mit einer Auswerteeinheit und/oder Steuereinheit der MR-PET-Vorrichtung ermöglicht. Dabei kann das Signalübertragungselement beispielsweise ein Kabel usw. sein.

Das Übertragungselement der Motoreinheit umfasst bevorzugt einen Seilzug. Dabei kann die maximale Verschiebung des zumindest einen Steckverbindungselements innerhalb der Führungseinheit durch beispielsweise Knoten im Seilzug begrenzt werden. Alternativ kann auch die maximale Verschiebung des zumindest einen Steckverbindungselements innerhalb des Führungselements durch beispielsweise einen Anschlag innerhalb des Führungselements begrenzt werden. Bevorzugt umfasst die maximale Verschiebung des zumindest einen Steckverbindungselements 40 cm. Bevorzugt umfasst die maximale Verschiebung des zumindest einen Steckverbindungselements 35 cm. Bevorzugt umfasst die maximale Verschiebung des zumindest einen Steckverbindungselements 30 cm. Bevorzugt umfasst die maximale Verschiebung des zumindest einen Steckverbindungselements 25 cm. Bevorzugt umfasst die maximale Verschiebung des zumindest einen Steckverbindungselements 20 cm. Die minimale Verschiebung des zumindest einen Steckverbindungselements resultiert aus der Auflösung des PET-Detektors.

Hierdurch kann vorteilhaft einfache Verschiebung und/oder Positionierung des zumindest einen Steckverbindungselements in seine Zielposition ermöglicht werden und damit außerhalb des FOVs und/oder des Strahlengangs der PET-Vorrichtung während einer MR-PET-Untersuchung angeordnet werden.

Erfindungsgemäß kann es vorgesehen sein, dass die Einstelleinheit ein Führungselement aufweist, in dem das zumindest eine Steckverbindungselement der Steckverbindungseinheit bewegbar gelagert ist, wobei die Motoreinheit ein Übertragungselement aufweist, das zu einer Einstellung von zumindest zwei unterschiedlichen Zielpositionen des zumindest einen Steckverbindungselements innerhalb des Führungselements ausgebildet ist. Die zumindest zwei unterschiedlichen Zielpositionen können dabei vorgegebene Zielpositionen umfassen, die beispielsweise durch das Führungselement und/oder durch das Übertragungselement vorgegeben werden. Beispielweise kann das Übertragungselement einen Seilzug umfassen, wobei die beiden Zielpositionen durch Knoten im Seilzug festgelegt werden können. Bevorzugt umfassen die beiden unterschiedlichen Zielpositionen zwei Endpositionen für das zumindest eine Steckverbindungselement, die bevorzugt maximal 40 cm voneinander entfernt sind. Bevorzugt umfassen die beiden unterschiedlichen Zielpositionen zwei Endpositionen für das zumindest eine Steckverbindungselement, die bevorzugt maximal 35 cm voneinander entfernt sind. Bevorzugt umfassen die beiden unterschiedlichen Zielpositionen zwei Endpositionen für das zumindest eine Steckverbindungselement, die bevorzugt maximal 30 cm voneinander entfernt sind. Bevorzugt umfassen die beiden unterschiedlichen Zielpositionen zwei Endpositionen für das zumindest eine Steckverbindungselement, die bevorzugt maximal 25 cm voneinander entfernt sind. Besonders vorteilhaft umfassen die beiden unterschiedlichen Zielpositionen zwei Endpositionen für das zumindest eine Steckverbindungselement, die bevorzugt maximal 20 cm voneinander entfernt sind. Derart kann besonders einfach und zuverlässig das zumindest eine Steckverbindungselement außerhalb des FOVs und/oder des Strahlengangs der PET-Vorrichtung während einer MR-PET-Untersuchung angeordnet werden. Zudem kann derart eine besonders einfache und kostengünstige Einstelleinheit für die Einstellung der Zielpositionen des zumindest einen Steckverbindungselements zur Verfügung gestellt werden.

Erfindungsgemäß ist vorgesehen, dass die Motoreinheit einen Kraftsensor aufweist zur Erfassung einer auf das Übertragungselement wirkenden Zugkraft. Der Kraftsensor kann beispielsweise ein Dehnmessstreifen und/oder weitere, dem Fachmann als sinnvoll erscheinende Kraftsensoren zur Erfassung der auf das Übertragungselement wirkenden Zugkraft aufweisen. Bei einer Positionierung des zumindest einen Steckverbindungselements sollte eine auf das Übertragungselement wirkende Zugkraft im Wesentlichen konstant sein. Ist jedoch ein Hindernis vorhanden, dass ein Verschieben des zumindest einen Steckverbindungselement erschwert, erhöht sich somit eine auf das Übertragungselement wirkende Zugkraft. Beispielsweise kann bei einer gesteckten lokalen Hochfrequenzspule das Kabel zwischen der lokalen Hochfrequenzspule und dem Steckverbindungselement um den Patienten gewickelt sein, was zu Verletzung des Patienten führen kann. Zudem kann es auch sein, dass die Position des Patienten einen Teilbereich der Führungsschiene abdeckt. Durch den Kraftsensor kann ein Vorhandensein eines derartigen Hindernisses erfasst werden. Dies ermöglicht auch ein Stoppen des Positionierungsvorgans des zumindest einen Steckverbindungselements in die Zielposition, so dass eine Verletzung des Patienten und/oder eine Beschädigung von Bauteilen verhindert werden kann. Hierdurch kann ein hoher Sicherheitsstandard bei einer automatischen Einstellung der Zielposition des zumindest einen Steckverbindungselements bereitgestellt werden.

In einer vorteilhaften Weiterbildung der erfindungsgemäßen MR-PET-Vorrichtung kann es vorgesehen sein, dass die Einstelleinheit eine Positionserfassungseinheit, die zu einer Erfassung einer Position des zumindest einen Steckverbindungselements ausgebildet ist. Eine derartige Positionserfassungseinheit kann im einfachsten Fall, bei dem beispielsweise nur zwei Positionen zur Einstellung der Zielposition des zumindest einen Steckverbindungselements zur Verfügung stehen, zwei Lichtschranken umfassen. Zudem kann die Positionserfassungseinheit auch einen magnetresonanzkompatiblen Encoder, der zur Erfassung einer absoluten Position und/oder Geschwindigkeit und/oder Richtung der Bewegung des zumindest einen Steckverbindungselements ausgebildet ist, umfassen. Bevorzugt umfasst der Encoder einen optischen Encoder. Zudem sind weitere, dem Fachmann als sinnvoll erscheinende Positionserfassungseinheiten jederzeit denkbar. Hierdurch kann eine einfache und automatische Erfassung der Position, insbesondere eine aktuelle Position, des zumindest einen Steckverbindungselements erreicht werden. Insbesondere kann hierbei die Position bereits vor der Einstellung der Zielposition des zumindest einen Steckverbindungselements als auch die Zielposition des zumindest einen Steckverbindungselements erfasst werden.

In einer vorteilhaften Weiterbildung der erfindungsgemäßen MR-PET-Vorrichtung kann es vorgesehen sein, dass die Einstelleinheit ein Führungselement aufweist, in dem das zumindest eine Steckverbindungselement bewegbar gelagert ist, wobei die Positionserfassungseinheit an dem Führungselement angeordnet ist. Durch die Erfassung der aktuellen Position des zumindest einen Steckverbindungselements direkt am Führungselement und damit am zumindest einen Steckverbindungselement können vorteilhaft Ungenauigkeiten des Übertragungselements der Motoreinheit bei der Erfassung der Position des zumindest einen Steckverbindungselements verhindert werden. Umfasst das Übertragungselement beispielsweise einen Seilzug, könnte bei einer Erfassung der Position in der Nähe der Motoreinheit eine Dehnung des Seilzugs zu Ungenauigkeiten in der erfassten Position führen, was durch die Anordnung der Positionserfassungseinheit am Führungselement vorteilhaft verhindert ist.

In einer vorteilhaften Weiterbildung der erfindungsgemäßen MR-PET-Vorrichtung kann es vorgesehen sein, dass die Einstelleinheit eine Steuereinheit aufweist, die dazu ausgebildet ist, anhand von aktuellen Positionsdaten des zumindest einen Steckverbindungselements und anhand von zumindest einen Untersuchungsparameter eine Zielposition für das zumindest eine Steckverbindungselement zu ermitteln. Vorzugsweise wird die aktuelle Position des zumindest einen Steckverbindungselements durch die Positionserfassungseinheit bereitgestellt. Der zumindest eine Untersuchungsparameter kann beispielsweise eine Tischposition des Patiententischs, die dieser während der MR-PET-Untersuchung einnehmen soll, umfassen. Alternativ oder zusätzlich kann der zumindest eine Untersuchungsparameter auch eine Position und/oder eine Art und/oder eine Anzahl der mit dem zumindest einen Steckverbindungselement verbundenen externen Vorrichtungen, insbesondere lokalen Hochfrequenzspulen, umfassen. Alternativ oder zusätzlich kann der zumindest eine Untersuchungsparameter auch ein FOV und/oder eine Position des Strahlengangs der PET-Vorrichtung in Bezug auf eine Untersuchungsposition des Patiententischs und/oder weitere, dem Fachmann als sinnvoll erscheinende Parameter umfassen.

Die erfindungsgemäße Steuereinheit umfasst zumindest ein Rechenmodul und/oder einen Prozessor. So ist insbesondere die Steuereinheit dazu ausgebildet, computerlesbare Instruktionen auszuführen, um die Zielposition für das zumindest eine Steckverbindungselement zu ermitteln. Insbesondere umfasst die Steuereinheit eine Speichereinheit, wobei auf der Speichereinheit computerlesbare Informationen gespeichert sind, wobei die Steuereinheit dazu ausgebildet ist, die computerlesbaren Informationen von der Speichereinheit zu laden und die computerlesbaren Informationen auszuführen.

Die Komponenten der Steuereinheit können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Diese Ausgestaltung der Erfindung weist den Vorteil auf, dass besonders schnell und effektiv eine ermittelte Zielposition des zumindest einen Steckverbindungselement für automatische Einstellung der Zielposition bereitgestellt werden kann.

In einer vorteilhaften Weiterbildung der erfindungsgemäßen MR-PET-Vorrichtung kann es vorgesehen sein, dass die Steuereinheit dazu ausgebildet ist, eine automatische Einstellung der Zielposition des zumindest einen Steckverbindungselements zu steuern. Vorzugsweise ist hierbei die Steuereinheit zur Steuerung der Positionserfassungseinheit und der Einstelleinheit ausgebildet. Derart kann eine besonders schnelle und effektive Einstellung der Zielposition des zumindest einen Steckverbindungselement für die anstehende MR-PET-Untersuchung erreicht werden.

Des Weiteren geht die Erfindung aus von einem Verfahren zu einem automatischen Einstellen einer Zielposition zumindest eines Steckverbindungselements an einem Patiententisch mittels einer Einstelleinheit einer erfindungsgemässen MR-PET-Vorrichtung, umfassend die folgenden Verfahrensschritte:
- Bereitstellen einer aktuellen Positionsinformation des zumindest eines Steckverbindungselements,
- Bereitstellen zumindest eines Untersuchungsparameters,
- Ermitteln der Zielposition des zumindest eines Steckverbindungselements anhand der aktuellen Positionsinformation und des zumindest einen Untersuchungsparameters, und
- Einstellen der Zielposition des zumindest eines Steckverbindungselements.

Bevorzugt erfolgt das Bereitstellen der aktuellen Positionsinformation des zumindest eine Steckverbindungselements automatisch und/oder selbsttätig durch die Positionserfassungseinheit der Einstelleinheit. Die aktuelle Positionsinformation wird dabei vorteilhafterweise der Steuereinheit der Einstelleinheit bereitgestellt, wobei die Steuereinheit ein Erfassen der aktuellen Positionsinformation steuert.

Das Bereitstellen des zumindest einen Untersuchungsparameter erfolgt bevorzugt automatisch und/oder selbsttätig durch die Steuereinheit der Einstelleinheit. Hierbei kann die Steuereinheit auf in der MR-PET-Vorrichtung hinterlegten Untersuchungsparameter zugreifen. Der zumindest eine Untersuchungsparameter kann beispielsweise eine Tischposition des Patiententischs, die dieser während der MR-PET-Untersuchung einnehmen soll, umfassen. Alternativ oder zusätzlich kann der zumindest eine Untersuchungsparameter auch eine Position und/oder eine Art und/oder eine Anzahl der mit dem zumindest einen Steckverbindungselement verbundenen externen Vorrichtungen, insbesondere lokalen Hochfrequenzspulen, umfassen. Alternativ oder zusätzlich kann der zumindest eine Untersuchungsparameter auch ein FOV und/oder eine Position des Strahlengangs der PET-Vorrichtung in Bezug auf eine Untersuchungsposition des Patiententischs und/oder weitere, dem Fachmann als sinnvoll erscheinende Parameter umfassen.

Das Ermitteln des Zielposition des zumindest einen Steckverbindungselements erfolgt bevorzugt automatisch und/oder selbsttätig mittels der Steuereinheit der Einstelleinheit. Bevorzugt umfasst hierbei die Steuereinheit eine entsprechende Software und/oder Algorithmus zur Bestimmung der Zielposition für zumindest ein Steckverbindungselement.

Das Einstellen der Zielposition des zumindest einen Steckverbindungselements erfolgt bevorzugt automatisch und/oder selbsttätig mittels der Einstelleinheit, wobei die Steuereinheit der Einstelleinheit hierzu die Motoreinheit der Einstelleinheit entsprechend ansteuert.

Durch die Erfindung kann vorteilhaft das Steckverbindungselement besonders zeitsparend in seine Zielposition bewegt werden und damit außerhalb des FOV der PET-Vorrichtung angeordnet und/oder positioniert werden. Damit einhergehend können unerwünschte Artefakte und/oder Störeinflüsse in den PET-Bilddaten minimiert werden und auch eine Bildqualität vorteilhaft erhöht werden.

Gegenüber einer manuellen Einstellung, die vor einem Einfahren des Patienten in den Patientenaufnahmebereich erfolgt, bei der der Benutzer die Zielposition nur grob abschätzen kann, kann hierbei die automatische Einstellung der Zielposition des zumindest einen Steckverbindungselements auch erst erfolgen, wenn der zu untersuchende Bereich bereits im FOV der MR-PET-Vorrichtung angeordnet ist. Hingegen kann es bei einer manuellen Einstellung der Zielposition des zumindest einen Steckverbindungselements vorkommen, dass hierzu der Patient mehrmals aus dem Patientenaufnahmebereich der MR-PET-Vorrichtung hinein und wieder herausgefahren wird und jedes Mal die Position des zumindest einen Steckverbindungselements neu eingestellt werden muss, bis diese nicht mehr in dem FOV insbesondere der PET-Vorrichtung angeordnet ist, was jedoch sehr zeitaufwendig ist.

Ein weiterer Vorteil ist, dass aufgrund der zeitsparenden Positionierung des zumindest einen Steckerelements ein Zerfall eines Tracers und/oder eines Radiopharmakons bereits vor Beginn der MR-PET-Untersuchung verhindert wird und damit eine zusätzlichen Strahlenexposition im Patienten vermieden wird.

Die Vorteile des erfindungsgemäßen Verfahrens entsprechen im Wesentlichen den Vorteilen der erfindungsgemäßen MR-PET-Vorrichtung, welche vorab im Detail ausgeführt sind. Hierbei erwähnte Merkmale, Vorteile oder alternative Ausführungsformen können ebenso auf die anderen beanspruchten Gegenstände übertragen werden und umgekehrt.

In einer vorteilhaften Weiterbildung des erfindungsgemäßen Verfahrens kann es vorgesehen sein, dass vor dem Einstellen der Zielposition des zumindest einen Steckverbindungselements eine Sicherheitsabfrage generiert wird und mittels einer Benutzerschnittstelle an einen Benutzer ausgegeben wird. Die Sicherheitsabfrage kann beispielsweise eine Information über die Zielposition umfassen und dem Benutzer mitteilen, dass durch eine Bestätigungseingabe eine Einstellung und/oder Positionierung des zumindest einen Steckverbindungselements in diese Zielposition gestartet wird.

Die Benutzerschnittstelle umfasst bevorzugt eine Ausgabeeinheit, insbesondere eine optische Ausgabeeinheit, wie beispielsweise einen Monitor und/oder ein Display. Des Weiteren umfasst die Benutzerschnittstelle auch eine Eingabeeinheit, wie beispielsweise eine Tastatur und/oder eine Computermaus, umfassen.

Derart kann ein hoher Sicherheitsstandard für die Einstellung und/oder Positionierung des zumindest einen Steckverbindungselements in diese Zielposition bereitgestellt werden. Insbesondere kann derart der Benutzer vorher nochmals überprüfen, ob die ermittelte Zielposition des zumindest einen Steckverbindungselements außerhalb des FOVs der PET-Vorrichtung angeordnet ist und/oder mit der geplanten MR-PET-Untersuchung übereinstimmt.

In einer vorteilhaften Weiterbildung des erfindungsgemäßen Verfahrens kann es vorgesehen sein, dass das Einstellen der Zielposition des zumindest einen Steckverbindungselements von einer Kamera erfasst wird und mittels der Benutzerschnittstelle an einen Benutzer ausgegeben wird. Bevorzugt ist die Kamera derart angeordnet, dass ein Sichtfeld der Kamera das FOV der MR-PET-Vorrichtung und insbesondere das zumindest eine Steckverbindungselement und die Führungseinheit, in der das zumindest eine Steckverbindungselement bewegbar gelagert ist, erfasst wird. Hierzu kann die Kamera innerhalb des Patientenaufnahmebereichs oder auch außerhalb des Patientenaufnahmebereichs angeordnet sein.

Diese Ausgestaltung der Erfindung ermöglicht eine vorteilhafte Überwachung der Einstellung der Zielposition durch den Benutzer. Insbesondere kann hierbei der Benutzer bei unerwarteten Hindernissen oder Zwischenfällen während des Einstellens der Zielposition eingreifen und damit auch Verletzungen des Patienten und/oder Beschädigungen von Hardware-Komponenten vorteilhaft verhindern.

In einer vorteilhaften Weiterbildung des erfindungsgemäßen Verfahrens kann es vorgesehen sein, dass während des Einstellens der Zielposition des zumindest einen Steckverbindungselements der Einstellvorgangs manuell beendbar ist. Bevorzugt weist hierbei die MR-PET-Vorrichtung, insbesondere die Benutzerschnittstelle, einen Not-Aus-Schalter auf. Der Not-Aus-Schalter kann beispielsweise ein Pop-up-Fenster umfassen, das während des Einstellvorgangs für den Benutzer mittels der Benutzerschnittstelle angezeigt wird. Dieses Pop-up-Fenster kann dabei einen Not-Aus-Button umfassen. Bevorzugt wird ein derartige Pop-up-Fenster automatisch von der Steuereinheit generiert und bereitgestellt.

Diese Ausgestaltung der Erfindung weist den Vorteil auf, dass der Benutzer bei unerwarteten Hindernissen oder Zwischenfällen während des Einstellens der Zielposition des zumindest einen Steckverbindungselements eingreifen kann und damit auch Verletzungen des Patienten und/oder Beschädigungen von Hardware-Komponenten vorteilhaft verhindern werden können.

Weitere Vorteile, Merkmale und Einzelheiten der Erfindung ergeben sich aus den im Folgenden beschriebenen Ausführungsbeispielen sowie anhand der Zeichnungen.

Es zeigen:
- Fig. 1: eine erfindungsgemäße MR-PET-Vorrichtung in einer schematischen Darstellung,
- Fig. 2: ein Patiententisch mit dem zumindest einen Steckverbindungselement und einer Einstelleinheit,
- Fig. 3: ein erstes Ausführungsbeispiel des erfindungsgemäßen Verfahrens zu einem automatischen Einstellen einer Zielposition zumindest eines Steckverbindungselements,
- Fig. 4: ein zweites Ausführungsbeispiel des erfindungsgemäßen Verfahrens zu einem automatischen Einstellen einer Zielposition zumindest eines Steckverbindungselements, und
- Fig. 5: ein alternatives Ausführungsbeispiel der Steckverbindungseinheit.

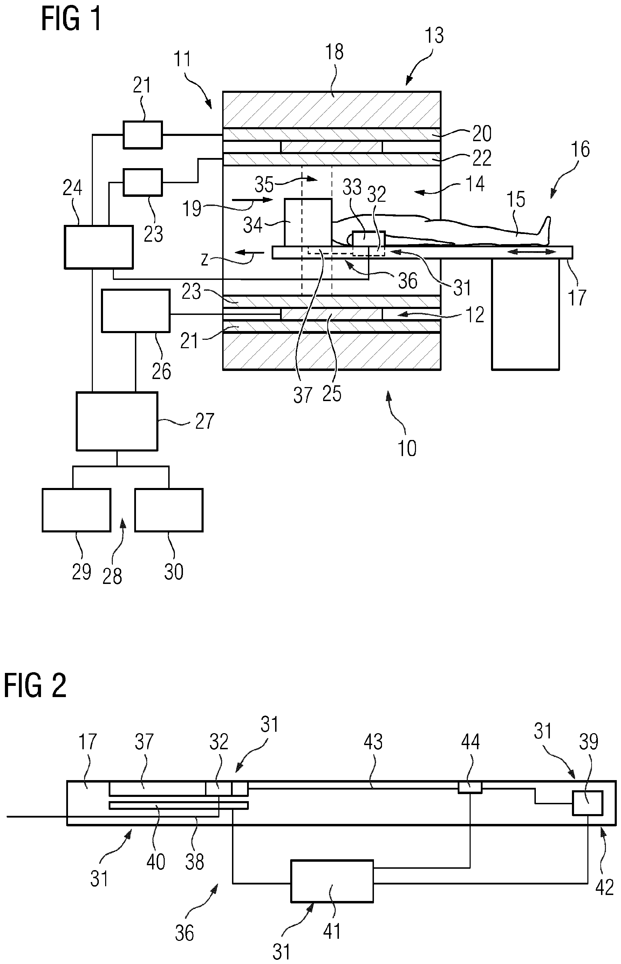

Fig. 1 zeigt eine Ausführungsform einer erfindungsgemäßen MR-PET-Vorrichtung 10 in einer schematischen Darstellung. Die MR-PET-Vorrichtung 10 umfasst eine Magnetresonanzvorrichtung 11 und eine Positronen-Emissions-Tomographie-Vorrichtung (PET-Vorrichtung 12).

Die Magnetresonanzvorrichtung 11 umfasst eine von einer Magneteinheit gebildeten Scannereinheit 13 und einen von der Scannereinheit 13 umgebenen Patientenaufnahmebereich 14 zu einer Aufnahme eines Patienten 15. Der Patientenaufnahmebereich 14 im vorliegenden Ausführungsbeispiel ist zylinderförmig ausgebildet und in einer Umfangsrichtung von der Scannereinheit 13, insbesondere von der Magneteinheit, zylinderförmig umgeben. Der Patient 15 kann mittels einer Patientenlagerungsvorrichtung 16 der MR-PET-Vorrichtung 10 in den Patientenaufnahmebereich 14 geschoben und/oder gefahren werden. Die Patientenlagerungsvorrichtung 16 weist hierzu einen innerhalb des Patientenaufnahmebereichs 14 bewegbar ausgestalteten Patiententisch 17 auf. Insbesondere ist hierbei der Patiententisch 17 in Richtung einer Längserstreckung des Patientenaufnahmebereichs 14 und/oder in z-Richtung bewegbar gelagert.

Die Scannereinheit 13, insbesondere die Magneteinheit, umfasst einen supraleitenden Grundmagneten 18 zu einem Erzeugen eines starken und insbesondere konstanten Grundmagnetfelds 19. Weiterhin weist die Scannereinheit 13, insbesondere die Magneteinheit, eine Gradientenspuleneinheit 20 zu einer Erzeugung von Magnetfeldgradienten auf, die für eine Ortskodierung während einer Bildgebung verwendet werden. Die Gradientenspuleneinheit 20 wird mittels einer Gradientensteuereinheit 21 der Magnetresonanzvorrichtung 11 gesteuert. Die Scannereinheit 13, insbesondere die Magneteinheit, umfasst weiterhin eine Hochfrequenzantenneneinheit 22 zu einer Anregung einer Polarisation, die sich in dem von dem Grundmagneten 18 erzeugten Grundmagnetfeld 19 einstellt. Die Hochfrequenzantenneneinheit 22 wird von einer Hochfrequenzantennensteuereinheit 23 der Magnetresonanzvorrichtung 11 gesteuert und strahlt hochfrequente Magnetresonanzsequenzen in den Patientenaufnahmebereich 14 der Magnetresonanzvorrichtung 11 ein.

Zu einer Steuerung des Grundmagneten 18, der Gradientensteuereinheit 20 und zur Steuerung der Hochfrequenzantennensteuereinheit 22 weist die Magnetresonanzvorrichtung 11 eine Magnetresonanz-Steuereinheit 24 auf. Die Magnetresonanz-Steuereinheit 24 steuert zentral die Magnetresonanzvorrichtung 11, wie beispielsweise das Durchführen einer vorbestimmten bildgebenden Gradientenechosequenz. Zudem umfasst die Magnetresonanz-Steuereinheit 24 eine nicht näher dargestellte Auswerteeinheit zu einer Auswertung von medizinischen Magnetresonanzbilddaten.

Die dargestellte Magnetresonanzvorrichtung 11 kann selbstverständlich weitere Komponenten umfassen, die Magnetresonanzvorrichtungen 11 gewöhnlich aufweisen. Eine allgemeine Funktionsweise einer Magnetresonanzvorrichtung 11 ist zudem dem Fachmann bekannt, so dass auf eine detaillierte Beschreibung der allgemeinen Komponenten verzichtet wird.

Die PET-Vorrichtung umfasst mehrere Positronen-Emissions-Tomographie-Detektormodule (PET-Detektormodule 25), die zu einer Ringform angeordnet sind und den Patientenaufnahmebereich 14 in der Umfangsrichtung umgeben. Die PET-Detektormodule 25 weisen jeweils mehrere, nicht näher dargestellte Positronen-Emissions-Tomographie-Detektorelemente (PET-Detektorelemente) auf, die zu einem PET-Detektorarray angeordnet sind, das ein Szintillationsdetektorarray mit Szintillationskristallen, beispielsweise LSO-Kristalle, umfasst. Des Weiteren umfassen die PET-Detektormodule 25 jeweils ein Photodiodenarray, beispielsweise Avalanche-Photodiodenarray oder APD-Photodiodenarray, die dem Szintillationsdetektorarray nachgeschaltet innerhalb der PET-Detektormodule 25 angeordnet sind.

Mittels der PET-Detektormodule 25 werden Photonenpaare, die aus der Annihilation eines Positrons mit einem Elektron resultieren, erfasst. Trajektorien der beiden Photonen schlie-ßen einen Winkel von 180° ein. Zudem weisen die beiden Photonen jeweils eine Energie von 511 keV auf. Das Positron wird hierbei von einem Radiopharmakon emittiert, wobei das Radiopharmakon über eine Injektion dem Patienten 15 verabreicht wird. Beim Durchlaufen von Materie im Strahlengang können die bei der Annihilation entstandenen PET-Photonen abgeschwächt werden, wobei die Abschwächungswahrscheinlichkeit von der Pfadlänge durch die Materie und dem entsprechenden Abschwächungskoeffizienten der Materie abhängt.

Zudem weisen die PET-Detektormodule 25 jeweils eine Detektorelektronik auf, die eine elektrische Verstärkerschaltung und weitere, nicht näher dargestellte Elektronikkomponenten umfasst.

Zu einer Steuerung der Detektorelektronik und der PET-Detektormodule 25 weist die MR-PET-Vorrichtung 10, insbesondere die PET-Vorrichtung 12, eine PET-Steuereinheit 26 auf. Die PET-Steuereinheit 26 steuert zentral die PET-Vorrichtung 12. Zudem umfasst die PET-Steuereinheit 26 eine Auswerteeinheit zu einer Auswertung von PET-Daten. Die dargestellte PET-Vorrichtung 12 kann selbstverständlich weitere Komponenten umfassen, die PET-Vorrichtungen 12 gewöhnlich aufweisen. Eine allgemeine Funktionsweise einer PET-Vorrichtung 12 ist zudem dem Fachmann bekannt, so dass auf eine detaillierte Beschreibung der allgemeinen Komponenten verzichtet wird.

Die MR-PET-Vorrichtung 10 weist zudem eine zentrale Recheneinheit 27 auf, die beispielsweise eine Erfassung und/oder eine Auswertung von Magnetresonanzsignalen und von PET-Signalen aufeinander abstimmt. Die Recheneinheit 27 kann eine zentrale Systemsteuereinheit sein.

Des Weiteren umfasst die MR-PET-Vorrichtung 10 eine Benutzerschnittstelle 28, die mit der zentralen Recheneinheit 27 verbunden ist. Steuerinformationen wie beispielsweise Bilddaten können auf einer Ausgabeeinheit 29, beispielsweise auf zumindest einem Monitor, der Benutzerschnittstelle 28 für ein medizinisches Bedienpersonal angezeigt werden. Weiterhin weist die Benutzerschnittstelle 28 eine Eingabeeinheit 30 auf, mittels der Informationen und/oder Parameter während eines Messvorgangs von dem medizinischen Bedienpersonal eingegeben werden können.

Der Patiententisch 17 weist zudem eine Steckverbindungseinheit 31 auf, wobei die Steckverbindungseinheit 31 zumindest ein Steckverbindungselement 32 aufweist, das am Patiententisch 17 angeordnet ist. Die Steckverbindungeinheit 31 kann dabei zwei oder mehr Steckverbindungselemente 32 aufweisen. Vorliegend ist die Erfindung anhand eines einzigen Steckverbindungselements 32 näher erläutert. Das Steckverbindungselement 32 ist dazu ausgebildet, mit einem zu dem Steckverbindungselement 32 der Steckverbindungseinheit 31 korrespondierenden Steckverbindungselement 33 einer externen Vorrichtung 34 eine lösbare Verbindung einzugehen. Die externe Vorrichtung 34 umfasst beispielsweise eine Zubehöreinheit, wie insbesondere eine lokale Hochfrequenzspule.

Das Steckverbindungselement 32 der Steckverbindungseinheit 31 ist bevorzugt als Steckerbuchse ausgebildet. Das Steckverbindungselement 33 der externen Vorrichtung 34 ist als dazu korrespondierender Stecker ausgebildet.

Für eine MR-PET-Untersuchung wird der Patiententisch 17 zusammen mit dem Patienten 15 und den am Patienten 15 und/oder am Patiententisch 17 angeordneten und/oder positionierten weiteren Einheiten, wie beispielsweise einer lokalen Hochfrequenzspule in den Patientenaufnahmebereich 14 eingefahren, bis der zu untersuchende Bereich des Patienten 15 im FOV der MR-PET-Vorrichtung angeordnet ist. Dabei kann es auch sein, dass das Steckverbindungselement 32 der Steckverbindungseinheit 31 innerhalb eines FOVs 35 der PET-Vorrichtung 12 angeordnet ist. Um dies zu verhindern, ist eine Position des Steckverbindungselements 32 der Steckverbindungseinheit 31 einstellbar ausgebildet. Hierzu weist die Steckverbindungseinheit 31 eine Einstelleinheit 36 auf, die zu einer selbsttätigen und/oder automatischen Einstellung einer Zielposition des Steckverbindungselements 32 am Patiententisch 17 ausgebildet ist.

In Fig. 2 ist der Patiententisch 17 mit der Steckverbindungseinheit 31, insbesondere dem Steckverbindungselement 32 und der Einstelleinheit 36, näher dargestellt.

Für eine einstellbare Zielposition des Steckverbindungselements 31 der Steckverbindungseinheit 31 weist die Einstelleinheit 36 ein Führungselement 37 auf, in dem das Steckverbindungselement 32 bewegbar gelagert ist. Das Führungselement 37 ist als Führungsschiene und/oder als Führungsrinne ausgebildet, in der das Steckverbindungselement 32 in eine Richtung, bevorzugt in Längsrichtung des Patiententischs 17, bewegbar gelagert ist. Das Führungselement 37 kann dabei einen T-förmigen und/oder schwalbenschwanzförmigen Querschnitt aufweisen, um eine sichere Führung des Steckverbindungselements 32 zu gewährleisten. Zudem weist die Einstelleinheit 36 ein Kontaktelement 38 und/oder Signalübertragungselement auf, das Signale und/oder Daten zwischen dem bewegbar gelagerten Steckverbindungselement 32 zu einer Auswerteeinheit der Magnetresonanz-Steuereinheit 24 überträgt. Das Kontaktelement 38 kann dabei ein aufgewickeltes Kabel und/oder ein Spiralkabel umfassen, dass eine Bewegung des Steckverbindungselements 32 ausgleichen kann. Zudem kann das Kontaktelement 38 eine Kontaktplatte, die innerhalb des Führungselements 37 angeordnet ist, und/oder weitere, dem Fachmann als sinnvoll erscheinende Kontaktelemente 38 umfassen.

Für die selbsttätige und/oder automatische Einstellung der Zielposition des Steckverbindungselements 32 weist die Einstelleinheit 36 zudem eine Motoreinheit 39, eine Positionserfassungseinheit 40 und eine Steuereinheit 41 auf. Die Motoreinheit 39 ist bevorzugt magnetresonanzkompatibel ausgebildet. Beispielsweise kann die Motoreinheit 39 einen magnetresonanzkompatiblen Schrittmotor, wie er in der Patentanmeldung DE 10 2020 211 326 A1 oder der Patentanmeldung DE 10 2020 211 327 A1 beschrieben ist, umfassen. Alternativ oder zusätzlich kann die Motoreinheit 39 auch einen pneumatische Schrittmotor und/oder einen Piezo-Schrittmotor und/oder elektrischen Schrittmotor umfassen. Zudem ist die Motoreinheit 39 an einem Fußende 42 des Patiententischs 17 angeordnet, wobei die Motoreinheit 39 hierbei bevorzugt einen pneumatische Schrittmotor und/oder einen Piezo-Schrittmotor und/oder elektrischen Schrittmotor umfasst. Die Einstelleinheit 36, insbesondere die Motoreinheit 39, weist zudem ein Übertragungselement 43 auf, das das von der Motoreinheit 39 generierte Antriebsmoment auf das Steckverbindungselement 32 überträgt bzw. die Motoreinheit 39 mit dem Steckverbindungselement 32 verbindet. Das Übertragungselement 43 umfasst bevorzugt einen Seilzug für eine besonders exakte Einstellung einer Position des zumindest einen Steckverbindungselement 32.

Das Übertragungselement 43 ist zu einem Verschieben des Steckverbindungselements 32 in die Zielposition um mindestens 1,5 mm und um maximal 40 cm ausgebildet. Bevorzugt umfasst die maximale Verschiebung des zumindest einen Steckverbindungselements 32 35cm. Bevorzugt umfasst die maximale Verschiebung des zumindest einen Steckverbindungselements 32 30cm. Bevorzugt umfasst die maximale Verschiebung des zumindest einen Steckverbindungselements 32 25cm. Bevorzugt umfasst die maximale Verschiebung des zumindest einen Steckverbindungselements 32 20cm. Die minimale Verschiebung des Steckverbindungselements 32 resultiert aus der Auflösung der PET-Detektormodule 25.

Zudem kann Übertragungselement 43 auch zu einer Einstellung von zumindest zwei unterschiedlichen Zielpositionen des Steckverbindungselements 32 innerhalb des Führungselements 37 ausgebildet sein. Umfasst beispielsweise das Übertragungselement 43 einen Seilzug, so können die beiden Zielpositionen durch Knoten im Seilzug festgelegt werden. Dabei können die beiden unterschiedlichen Zielpositionen zwei Endpositionen umfassen, die bevorzugt maximal 40 cm voneinander entfernt sind. Bevorzugt sind die beiden Zielpositionen, insbesondere die Endpositionen, maximal 35 cm voneinander entfernt angeordnet. Bevorzugt sind die beiden Zielpositionen, insbesondere die Endpositionen, maximal 30 cm voneinander entfernt angeordnet. Bevorzugt sind die beiden Zielpositionen, insbesondere die Endpositionen, maximal 25 cm voneinander entfernt angeordnet. Bevorzugt sind die beiden Zielpositionen, insbesondere die Endpositionen, maximal 20 cm voneinander entfernt angeordnet.

Die Motoreinheit 39 weist zudem einen Kraftsensor 44 auf zu einer Erfassung einer auf das Übertragungselement 43 wirkenden Zugkraft. Der Kraftsensor 44 umfasst beispielsweise einen Dehnmessstreifen. Mittels des Kraftsensors 44 kann erfasst werden, wenn bei einer Positionierung des Steckverbindungselements 32 in eine Zielposition ein Hindernis vorhanden ist, das eine erhöhte Zugkraft zur Bewegung des Steckverbindungselements 32 erfordern würde.

Die Positionserfassungseinheit 40 ist zu einer Erfassung einer Position des Steckverbindungselements 32 ausgebildet. Hierzu ist die Positionserfassungseinheit 40 an dem Führungselement 37 angeordnet. Die Positionserfassungseinheit 40 kann beispielsweise Lichtschranken umfassen, wie dies vorteilhaft ist, wenn die Einstelleinheit 36 zur Einstellung nur definierter, bevorzugt von zwei Zielpositionen ausgebildet ist. Ist dagegen die Einstelleinheit 36 zu einer Einstellung einer beliebigen Zielposition innerhalb der Führungseinheit 37 ausgebildet, umfasst die Positionserfassungseinheit 40 dagegen bevorzugt einen Encoder, insbesondere einen magnetresonanzkompatiblen Encoder, der zur Erfassung einer absoluten Position und/oder Geschwindigkeit und/oder Richtung der Bewegung des zumindest einen Steckverbindungselements 32 ausgebildet ist. Bevorzugt umfasst der magnetresonanzkompatible Encoder einen optischen Encoder.

Die Steuereinheit 41 der Einstelleinheit 36 ist dazu ausgebildet, anhand von aktuellen Positionsdaten des Steckverbindungselements 32 und anhand von zumindest einem Untersuchungsparameter eine Zielposition für das Steckverbindungselement 32 zu ermitteln. Die Steuereinheit 41 ist zudem dazu ausgebildet, eine automatische Einstellung der Zielposition des Steckverbindungselements 32 zu steuern. Hierzu weist die Steuereinheit 41 eine entsprechende Steuersoftware und/oder Auswertesoftware auf. Die entsprechende Steuersoftware und/oder Auswertesoftware kann dabei in einer Speichereinheit der Steuereinheit 41 gespeichert sein oder auch in einer externen Speichereinheit.

Die aktuellen Positionsdaten des Steckverbindungselements 32 werden von der Positionserfassungseinheit 40 der Steuereinheit 41 bereitgestellt. Der zumindest eine Untersuchungsparameter kann beispielsweise eine Tischposition des Patiententischs 17, die dieser während der MR-PET-Untersuchung einnehmen soll, umfassen. Alternativ oder zusätzlich kann der zumindest eine Untersuchungsparameter auch eine Position und/oder eine Art und/oder eine Anzahl der mit dem zumindest einen Steckverbindungselement 32 verbundenen externen Vorrichtungen 34, insbesondere lokalen Hochfrequenzspulen, umfassen. Alternativ oder zusätzlich kann der zumindest eine Untersuchungsparameter auch ein FOV 35 und/oder eine Position des Strahlengangs der PET-Vorrichtung 12 in Bezug auf eine Untersuchungsposition des Patiententischs 17 und/oder weitere, dem Fachmann als sinnvoll erscheinende Parameter umfassen.

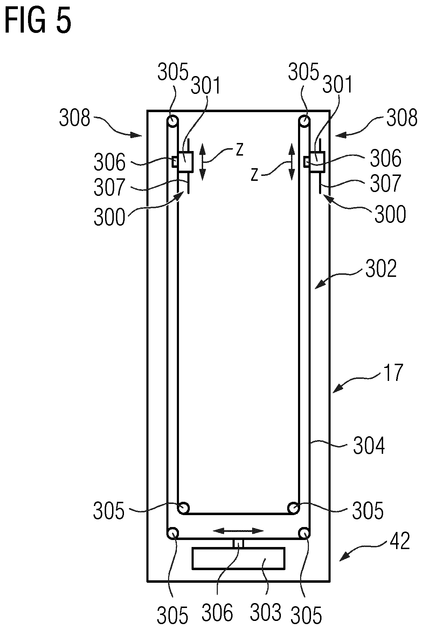

In Fig. 5 ist ein alternatives Ausführungsbeispiel der Steckverbindungseinheit 300 dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu dem Ausführungsbeispiel in den Fig. 1 und 2, wobei bezüglich gleichbleibender Bauteile, Merkmale und Funktionen auf die Beschreibung des Ausführungsbeispiels in den Fig. 1 und 2 verwiesen wird.

In Fig. 5 ist der Patiententisch 17 dargestellt mit einer Steckverbindungseinheit 300, wobei die Steckverbindungseinheit 300 zwei Steckverbindungselemente 301 und eine Einstelleinheit 302 aufweist. Jedes der beiden Steckverbindungselemente 301 ist zu einer lösbaren Verbindung mit einem korrespondierenden Steckverbindungselemente einer externen Vorrichtung, beispielsweise einer lokalen Hochfrequenzspule, ausgebildet. Die Einstelleinheit 302 weist eine Motoreinheit 303 und ein Übertragungselement 304 auf. Die Motoreinheit 303 ist am Fußende 42 des Patiententischs 17 angeordnet und umfasst im vorliegenden Ausführungsbeispiel einen pneumatischen Zylinder. Der pneumatische Zylinder ist beispielsweise aus einem Kunststoffmaterial gebildet. Zudem weist die Motoreinheit 303 eine Luftpumpe und zwei Luftventile auf für eine Umkehr einer Bewegungsrichtung einer Bewegung des mit mittels der Motoreinheit 303 bewegten und/oder angetriebenen Übertragungselements 304.

Das Übertragungselement 304 umfasst einen Seilzug, der mit den beiden Steckverbindungselementen 301 der Steckverbindungseinheit 300 verbunden ist. Die Einstelleinheit 302 weist zudem Rollen 305 auf, wobei der Seilzug ist mittels der Rollen 305, im vorliegenden Ausführungsbeispiel mittels sechs Rollen 305, gelagert und/oder geführt und/oder umgelenkt wird. Im vorliegenden Ausführungsbeispiel umfasst der Seilzug ein Seil aus Armidfasern. Grundsätzlich können auch andere Materialien zur Ausbildung des Seils des Seilzugs verwendet werden. Das Seil bildet hierbei eine geschlossene Schleife. Über einen Seilknoten 306 ist das Seil mit dem Zylinder der pneumatischen Motoreinheit 303 verbunden. Zudem ist das Seil über jeweils einen Seilknoten 306 mit den bewegbaren Steckverbindungselementen 301 verbunden.

Die Steckverbindungseinheit 300 weist zudem zwei Führungselemente 307 auf, die am Kopfende 308, insbesondere an einem seitlichen Bereich des Kopfendes 308, des Patiententischs 17 angeordnet sind. Die Führungselemente 307 weisen zudem jeweils eine in Fig. 5 nicht näher dargestellten Positionserfassungseinheit, beispielsweise einen Positionsencoder auf. Bei einer Bewegung des pneumatischen Zylinders wird über den Seilknoten 306 das Seil mitbewegt und über die beiden weiteren Seilknoten 306 die beiden Steckverbindungselemente 301 ebenfalls mitbewegt. Damit erfolgt auch eine Positionsverschiebung der beiden Steckverbindungselemente 301 in z-Richtung. Eine weitere Ausgestaltung der in Fig. 5 dargestellten Steckverbindungseinheit 300 entspricht den in Fig. 2 beschriebenen Ausführungen.

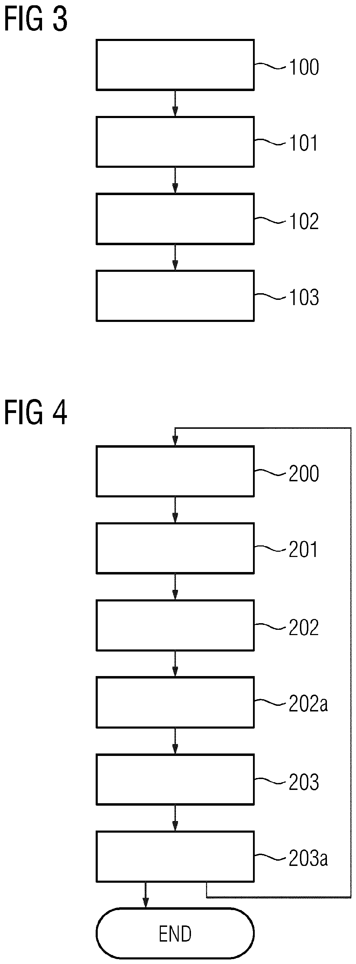

In Fig. 3 ist ein erstes Ausführungsbeispiel eines Verfahrens zu einem automatischen Einstellen einer Zielposition eines Steckverbindungselements 32 an einem Patiententisch 17 mittels einer Einstelleinheit 36 einer MR-PET-Vorrichtung 10 dargestellt. Die Einstelleinheit 36 ist hierbei gemäß den Ausführungen zu Fig. 2 ausgebildet.

In einem ersten Verfahrensschritt 100 erfolgt ein Bereitstellen einer aktuellen Positionsinformation des Steckverbindungselements 32. Das Bereitstellen der aktuellen Positionsinformation erfolgt mittels der Positionserfassungseinheit 40, die die aktuelle Position des Steckverbindungselements 32 erfasst. Insbesondere erfolgt das Bereitstellen der aktuellen Positionsinformation automatisch und/oder selbsttätig mittels der Positionserfassungseinheit 40, wobei die Positionserfassungseinheit 40 von der Steuereinheit 41 gesteuert wird. Die aktuelle Positionsinformation wird dabei von der Positionserfassungseinheit 40 der Steuereinheit 41 bereitgestellt.

In einem daran anschließenden zweiten Verfahrensschritt 101 erfolgt ein Bereitstellen zumindest eines Untersuchungsparameters. Der zumindest eine Untersuchungsparameter kann dabei Tischposition des Patiententischs 17, die dieser während der MR-PET-Untersuchung einnehmen soll, umfassen. Alternativ oder zusätzlich kann der zumindest eine Untersuchungsparameter auch eine Position und/oder eine Art und/oder eine Anzahl der mit dem zumindest einen Steckverbindungselement 32 verbundenen externen Vorrichtungen 34, insbesondere lokalen Hochfrequenzspulen, umfassen. Alternativ oder zusätzlich kann der zumindest eine Untersuchungsparameter auch ein FOV 35 und/oder eine Position des Strahlengangs der PET-Vorrichtung 12 in Bezug auf eine Untersuchungsposition des Patiententischs 17 und/oder weitere, dem Fachmann als sinnvoll erscheinende Parameter umfassen. Das Bereitstellen des zumindest einen Untersuchungsparameters erfolgt bevorzugt automatisch und/oder selbsttätig durch die Steuereinheit 41 der Einstelleinheit 36. Hierbei kann die Steuereinheit 41 auf in der MR-PET-Vorrichtung 10 hinterlegten Untersuchungsparameter zugreifen.

Anschließend erfolgt in einem weiteren, dritten Verfahrensschritt 102 ein Ermitteln der Zielposition des Steckverbindungselements 32 anhand der aktuellen Positionsinformation und des zumindest einen Untersuchungsparameters. Das Ermitteln der Zielposition erfolgt dabei automatisch und/oder selbsttätig mittels der Steuereinheit 41. Die Zielposition befindet sich dabei bevorzugt außerhalb eines Strahlengangs und/oder des FOVs 35 des PET-Detektors 12.

In einem daran anschließenden vierten Verfahrensschritt 103 erfolgt ein Einstellen der Zielposition des Steckverbindungselements 32. Insbesondere erfolgt das Einstellen der Zielposition des Steckverbindungselements 32 automatisch und/oder selbsttätig mittels der Motoreinheit 39, wobei die Motoreinheit 39 von der Steuereinheit 41 gesteuert wird.

In Fig. 4 ist ein alternatives Ausführungsbeispiel des Verfahrens zu einem automatischen Einstellen einer Zielposition eines Steckverbindungselements 32 an einem Patiententisch 17 mittels einer Einstelleinheit 36 einer MR-PET-Vorrichtung 10 dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu dem Ausführungsbeispiel in Fig. 3, wobei bezüglich gleichbleibender Bauteile, Merkmale und Funktionen auf die Beschreibung des Ausführungsbeispiels in Fig. 3 verwiesen wird.

In einem ersten Verfahrensschritt 200 erfolgt ein automatisches Bereitstellen einer aktuellen Positionsinformation des Steckverbindungselements 32, wie dies bereits in Fig. 3 zu dem Verfahrensschritt 100 beschrieben ist.

In einem daran anschließenden zweiten Verfahrensschritt 201 erfolgt ein automatisches Bereitstellen zumindest eines Untersuchungsparameters, wie dies bereits in Fig. 3 zu dem Verfahrensschritt 101 beschrieben ist.

In einem daran anschließenden dritten Verfahrensschritt 202 erfolgt ein Ermitteln einer Zielposition des Steckverbindungselements 32 anhand der aktuellen Positionsinformation und des zumindest einen Untersuchungsparameters, wie dies bereits in Fig. 3 zu dem Verfahrensschritt 102 beschrieben ist.

In einem daran anschließenden Verfahrensschritt 202a wird automatisch eine Sicherheitsabfrage generiert und mittels der Benutzerschnittstelle 28 an den Benutzer ausgegeben. Die Sicherheitsabfrage wird dabei automatisch von der Steuereinheit 41 generiert und an die Benutzerschnittstelle 28, insbesondere der Ausgabeeinheit 29, übermittelt und dort ausgegeben. Die Sicherheitsabfrage kann dem Benutzer die ermittelte Zielposition des Steckverbindungselements 32 mitteilen. Zudem umfasst die Sicherheitsabfrage eine Benutzereingabe, die der Benutzer tätigen muss, um einen anschließenden Positioniervorgang zu starten. Dies ermöglicht es dem Benutzer, den Positioniervorgang zu überwachen.

In einem daran anschließenden Verfahrensschritt 203 erfolgt ein automatisches Einstellen der Zielposition des Steckverbindungselements 32. Insbesondere erfolgt das Einstellen der Zielposition des Steckverbindungselements 32 automatisch und/oder selbsttätig mittels der Motoreinheit 39, wobei die Motoreinheit 39 von der Steuereinheit 41 gesteuert wird.

Das Einstellen der Zielposition des Steckverbindungselements 32 mittels der Motoreinheit 39 wird dabei in einem weiteren Verfahrensschritt 203a von einer Kamera erfasst und mittels der Benutzerschnittstelle 28, insbesondere der Ausgabeeinheit 29, an den Benutzer ausgegeben. Die Kamera kann hierbei von der Einstelleinheit 36 umfasst sein. Zudem kann hierzu auch eine Kamera genutzt werden, die bereits von der MR-PET-Vorrichtung 10 zur Patientenüberwachung genutzt wird. Das Erfassen der Einstellvorgangs der Zielposition des Steckverbindungselements 32 durch die Kamera wird hierbei von der Steuereinheit 41 gesteuert.

Des Weiteren wird in diesem Verfahrensschritt 203a des Erfassens der Zielposition des Steckverbindungselements 32 mittels der Kamera auch von der Steuereinheit 41 ein Eingabefenster, beispielweise ein Pop-up-Fenster generiert, das nur während des Einstellens der Zielposition des Steckverbindungselements 32 für den Benutzer angezeigt und/oder dargestellt wird. Dieses Pop-up-Fenster umfasst dabei einen Not-Aus-Schalter in Form eines Not-Aus-Buttons, der im Notfall vom Benutzer betätigt, beispielsweise angeklickt, werden kann. Durch Betätigen des Not-Aus-Buttons ist der Einstellvorgang vom Benutzer manuell beendbar. Dies ist von Vorteil, wenn beispielsweise der Benutzer ein Hindernis an der Zielposition des Steckverbindungselements 32 oder eine Notsituation des Patienten 15 in den Kameradaten erkennt usw.

Tätigt der Benutzer in dem Verfahrensschritt 203a nicht den Not-Aus-Button, ist nach dem Einstellvorgang des Einstellens des Steckverbindungselement 32 das Verfahren zu einem automatischen Einstellen einer Zielposition zumindest eines Steckverbindungselements 31 an einem Patiententisch 17 mittels einer Einstelleinheit 36 einer MR-PET-Vorrichtung 10 beendet.

Tätigt der Benutzer in dem Verfahrensschritt 203a den Not-Aus-Button, führt dies zu einem sofortigen Beenden des Einstellvorgangs des Einstellens des Steckverbindungselement 32. Nach einem Beseitigen des Hindernisses kann dann das Verfahren zu einem automatischen Einstellen einer Zielposition zumindest eines Steckverbindungselements 32 an einem Patiententisch 17 mittels einer Einstelleinheit 36 einer MR-PET-Vorrichtung 10 neu gestartet werden.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. MR-PET-Vorrichtung (10) mit einem Patiententisch (17) und einer Steckverbindungseinheit (31), die zumindest ein Steckverbindungselement (32), das am Patiententisch angeordnet ist, aufweist, wobei das zumindest eine Steckverbindungselement dazu ausgebildet ist, mit einem korrespondierenden Steckverbindungselement einer externen Vorrichtung (34) eine lösbare Verbindung einzugehen,
**dadurch gekennzeichnet, dass** eine Position des zumindest einen Steckverbindungselements (32) einstellbar ist und die Steckverbindungseinheit (31) eine Einstelleinheit (36) aufweist, die zu einer selbsttätigen Einstellung einer Zielposition des zumindest einen Steckverbindungselements (32) am Patiententisch (17) ausgelegt ist,
wobei die Einstelleinheit eine Motoreinheit (39), die mit dem zumindest einen Steckverbindungselement (32) verbunden ist, ein Führungselement (37), in dem das zumindest eine Steckverbindungselement der Steckverbindungseinheit bewegbar gelagert ist, ein Übertragungselement (43), das zu einer Einstellung von zumindest zwei unterschiedlichen Zielpositionen des zumindest einen Steckverbindungselements (32) innerhalb des Führungselements (37) ausgebildet ist, und einen Kraftsensor (44) aufweist zur Erfassung einer auf das Übertragungselement (43) wirkenden Zugkraft.

2. MR-PET-Vorrichtung nach Anspruch 1,
**dadurch gekennzeichnet, dass** die Motoreinheit (39) an einem Fußende des Patiententischs angeordnet ist.

3. MR-PET-Vorrichtung nach einem der vorhergehenden Ansprüche,
**dadurch gekennzeichnet, dass** die Einstelleinheit ein Führungselement aufweist, in dem das zumindest eine Steckverbindungselement der Steckverbindungseinheit bewegbar gelagert ist, wobei die Motoreinheit (39) ein Übertragungselement aufweist, das zu einem Verschieben des zumindest einen Steckverbindungselements um mindestens 1,5 mm und um maximal 40 cm innerhalb des Führungselements ausgebildet ist.

4. MR-PET-Vorrichtung nach einem der vorhergehenden Ansprüche,
**dadurch gekennzeichnet, dass** die Einstelleinheit eine Positionserfassungseinheit (40) aufweist, die zu einer Erfassung einer Position des zumindest einen Steckverbindungselements (32) ausgebildet ist.

5. MR-PET-Vorrichtung nach Anspruch 4,
**dadurch gekennzeichnet, dass** die Positionserfassungseinheit an dem Führungselement angeordnet ist.

6. MR-PET-Vorrichtung nach einem der vorhergehenden Ansprüche,
**dadurch gekennzeichnet, dass** die Einstelleinheit (36) eine Steuereinheit aufweist, die dazu ausgebildet ist, anhand von aktuellen Positionsdaten des zumindest einen Steckverbindungselements und anhand von zumindest einem Untersuchungsparameter eine Zielposition für das zumindest eine Steckverbindungselement zu ermitteln.

7. MR-PET-Vorrichtung nach Anspruch 6,
**dadurch gekennzeichnet, dass** die Steuereinheit (41) dazu ausgebildet ist, eine automatische Einstellung der Zielposition des zumindest einen Steckverbindungselements zu steuern.

8. Verfahren zu einem automatischen Einstellen einer Zielposition zumindest eines Steckverbindungselements an einem Patiententisch mittels einer Einstelleinheit einer MR-PET-Vorrichtung, wobei die MR-PET-Vorrichtung nach einem der Ansprüche 1 bis 7 ausgebildet ist, umfassend die folgenden Verfahrensschritte:
- Bereitstellen einer aktuellen Positionsinformation des zumindest einen Steckverbindungselements,
- Bereitstellen zumindest eines Untersuchungsparameters,
- Ermitteln der Zielposition des zumindest einen Steckverbindungselements anhand der aktuellen Positionsinformation und des zumindest einen Untersuchungsparameters, und
- Einstellen der Zielposition des zumindest einen Steckverbindungselements.

9. Verfahren nach Anspruch 8,
**dadurch gekennzeichnet, dass** vor dem Einstellen der Zielposition des zumindest einen Steckverbindungselements eine Sicherheitsabfrage generiert wird und mittels einer Benutzerschnittstelle an einen Benutzer ausgegeben wird.

10. Verfahren nach einem der Ansprüche 8 bis 9,
**dadurch gekennzeichnet, dass** das Einstellen der Zielposition des zumindest einen Steckverbindungselements von einer Kamera erfasst wird und mittels einer Benutzerschnittstelle an einen Benutzer ausgegeben wird.

11. Verfahren nach einem der Ansprüche 8 bis 10,
**dadurch gekennzeichnet, dass** während des Einstellens der Zielposition des zumindest einen Steckverbindungselements der Einstellvorgang manuell beendbar ist.

## Claims

1. MR-PET apparatus (10) with a patient table (17) and a plug-in connecting unit (31) having at least one plug-in connecting element (32) arranged on the patient table, wherein the at least one plug-in connecting unit is designed to enter into a releasable connection with a corresponding plug-in connecting element of an external apparatus (34),
**characterised in that** a position of the at least one plug-in connecting element (32) is adjustable and the plug-in connecting unit (31) has an adjusting unit (36) for an independent adjustment of a target position of the at least one plug-in connecting element (32) on the patient table (17),
wherein the adjusting unit has a motor unit (39) which is connected to the at least one plug-in connecting element (32), a guiding element (37) in which the at least one plug-in connecting element of the plug-in connecting unit is movably mounted, a transfer element (43) which is designed for an adjustment of at least two different target positions of the at least one plug-in connecting element (32) within the guiding element (37), and a force sensor (44) for acquiring a tensile force acting upon the transfer element (43).

2. MR-PET apparatus according to claim 1,
**characterised in that** the motor unit (39) is arranged at a foot end of the patient table.

3. MR-PET apparatus according to one of the preceding claims,
**characterised in that** the adjusting unit has a guiding element in which the at least one plug-in connecting element of the plug-in connecting unit is movably mounted, wherein the motor unit (39) has a transfer element which is designed for a displacement of the at least one plug-in connecting element by at least 1.5 mm and by not more than 40 cm within the guiding element.

4. MR-PET apparatus according to one of the preceding claims, **characterised in that** the adjusting unit has a position detection unit (40), which is designed for detecting a position of the at least one plug-in connecting element (32).

5. MR-PET apparatus according to claim 4,
**characterised in that** the position detection unit is arranged on the guiding element.

6. MR-PET apparatus according to one of the preceding claims,
**characterised in that** the adjusting unit (36) has a control unit which is designed, on the basis of current position data relating to the at least one plug-in connecting element and on the basis of at least one examination parameter, to establish a target position for the at least one plug-in connecting element.

7. MR-PET apparatus according to claim 6,
**characterised in that** the control unit (41) is designed to control an automatic adjustment of the target position of the at least one plug-in connecting element.

8. Method for an automatic adjustment of a target position of at least one plug-in connecting element on a patient table by means of an adjusting unit of an MR-PET apparatus, wherein the MR-PET apparatus is designed according to one of claims 1 to 7, comprising the following method steps:
- providing a current position information item of the at least one plug-in connecting element,
- providing at least one examination parameter,
- establishing the target position of the at least one plug-in connecting element on the basis of the current position information item and the at least one examination parameter, and
- adjusting the target position of the at least one plug-in connecting element.

9. Method according to claim 8,
**characterised in that** before the adjustment of the target position of the at least one plug-in connecting element, a safety query is generated and is output to a user by means of a user interface.

10. Method according to one of claims 8 to 9,
**characterised in that** the adjustment of the target position of the at least one plug-in connecting element is detected by a camera and is output to a user by means of a user interface.

11. Method according to one of claims 8 to 10,
**characterised in that** during the adjustment of the target position of the at least one plug-in connecting element, the adjustment process is able to be ended manually.

## Revendications

1. Installation (10) RM-PET comprenant une table (17) de patient et une unité (31) de connexion à fiche, qui a au moins un élément (32) de connexion à fiche monté sur la table de patient, dans laquelle le au moins un élément de connexion à fiche est constitué pour entrer dans une connexion détachable avec un élément de connexion à fiche correspondant d'une installation (34) extérieure,
**caractérisée en ce qu'**une position du au moins un élément (32) de connexion à fiche est réglable et l'unité (31) de connexion à fiche a une unité (36) de réglage, qui est conçue pour un réglage automatique d'une position cible du au moins un élément (32) de connexion à fiche sur la table (17) de patient,
dans laquelle l'unité de réglage a une unité (39) de moteur, qui est connectée au au moins un élément (32) de connexion à fiche, un élément (37) de guidage, dans lequel le au moins un élément de connexion à fiche de l'unité de connexion à fiche est monté mobile, un élément (43) de transmission, qui est constitué pour un réglage d'au moins deux positions cibles différentes du au moins un élément (32) de connexion à fiche dans l'élément (37) de guidage et un capteur (44) de force pour la détection d'une force de traction s'appliquant à l'élément (43) de transmission.

2. Installation RM-PET suivant la revendication 1,
**caractérisée en ce que** l'unité (39) de moteur est montée sur une extrémité de pied de la table de patient.

3. Installation RM-PET suivant l'une des revendications précédentes,
**caractérisée en ce que** l'unité de réglage a un élément de guidage, dans lequel le au moins un élément de connexion à fiche de l'unité de connexion à fiche est monté mobile, dans laquelle l'unité (39) de moteur a un élément de transmission, qui est constitué pour un déplacement du au moins un élément de connexion à fiche d'au moins 1,5 mm et au maximum de 40 cm dans l'élément de guidage.

4. Installation RM-PET suivant l'une des revendications précédentes,
**caractérisée en ce que** l'unité de réglage a une unité (40) de détection de position, qui est constituée pour la détection d'une position du au moins un élément (32) de connexion à fiche.

5. Installation RM-PET suivant la revendication 4,
**caractérisée en ce que** l'unité de détection de position est montée sur l'élément de guidage.

6. Installation RM-PET suivant l'une des revendications précédentes,
**caractérisée en ce que** l'unité (36) de réglage a une unité de commande, qui est constituée pour déterminer, à l'aide de données de position en cours du au moins un élément de connexion à fiche et à l'aide d'au moins un paramètre de recherche, une position cible du au moins un élément de connexion à fiche.

7. Installation RM-PET suivant la revendication 6,
**caractérisée en ce que** l'unité (41) de commande est constituée pour commander un réglage automatique de la position cible du au moins un élément de connexion à fiche.

8. Procédé de réglage automatique d'une position cible d'au moins un élément de connexion à fiche sur une table de patient au moyen d'une unité de réglage d'une installation RM-PET, dans lequel l'installation RM-PET est constituée suivant l'une des revendications 1 à 7, comprenant les stades de procédé suivants :
- on se procure une information sur la position en cours du au moins un élément de connexion à fiche,
- on se procure au moins un paramètre de recherche,
- on détermine la position cible du au moins un élément de connexion à fiche à l'aide de l'information sur la position en cours et du au moins un paramètre de recherche, et
- on règle la position cible du au moins un élément de connexion à fiche.

9. Procédé suivant la revendication 8,
**caractérisé en ce qu'**avant le réglage de la position cible du au moins un élément de connexion à fiche, on créé une demande de sécurité et on l'envoie à un utilisateur au moyen d'une interface d'utilisateur.

10. Procédé suivant l'une des revendications 8 à 9,
**caractérisé en ce que** l'on détecte le réglage de la position cible du au moins un élément de connexion à fiche par un appareil photographique et on l'envoie à un utilisateur au moyen d'une interface d'utilisateur.

11. Procédé suivant l'une des revendications 8 à 10,
**caractérisé en ce que**, pendant le réglage de la position cible du au moins un élément de connexion à fiche, on peut mettre fin manuellement à l'opération de réglage.
